(19) Europäisches Patentamt
European Patent Office
Office européen des brevets

(11) Veröffentlichungsnummer: **0 414 102 A2**

# (12) EUROPÄISCHE PATENTANMELDUNG

(21) Anmeldenummer: **90115565.5**

(22) Anmeldetag: **14.08.90**

(51) Int. Cl.5: **C07C 275/26**, C07D 303/16, C07C 305/06, C07C 309/10, C07F 9/38, C08G 18/38, C08G 18/66

(30) Priorität: **20.08.89 DE 3927630**

(43) Veröffentlichungstag der Anmeldung: **27.02.91 Patentblatt 91/09**

(84) Benannte Vertragsstaaten: **DE FR GB NL**

(71) Anmelder: **BASF Aktiengesellschaft**
**Carl-Bosch-Strasse 38**
**D-6700 Ludwigshafen(DE)**

(72) Erfinder: **Keppeler, Uwe, Dr.**
**Ungsteiner Strasse 22**
**D-6700 Ludwigshafen(DE)**
Erfinder: **Bobrich, Michael, Dr.**
**In den Muehlgaerten 3**
**D-6737 Boehl-Iggelheim(DE)**

## (54) Heterofunktionelle Diole und daraus hergestellte Polyurethanelastomere.

(57) Die Erfindung betrifft heterofunktionelle Diole der Formel (I) sowie daraus hergestellte Polyurethanelastomere.

EP 0 414 102 A2

## HETEROFUNKTIONELLE DIOLE UND DARAUS HERGESTELLTE POLYURETHANELASTOMERE

Die Erfindung betrifft heterofunktionelle Diole sowie daraus hergestellte Polyurethanelastomere.

Der Einsatz von Diolen zur Herstellung von Kondensations- oder Additionspolymeren, wie Polyester oder Polyurethane, die bevorzugt im Lacksektor eingesetzt werden, ist bekannt. Der Bedarf an Lacken für spezielle Anwendungsgebiete und mit gezielt ausgewählten Eigenschaften erfordert besondere Komponenten für deren Herstellung.

Aufgabe der Erfindung war es nun, Diole bereitzustellten, mit deren Hilfe sich Kondensations- und Additionspolymere, wie z.B. Polyester- oder Polyurethanelastomere mit funktionalisierten Seitenketten herstellen lassen, wobei die Anwesenheit der funktionellen Gruppen zu Polymeren mit speziellen Eigenschaften, wie z.B. Strahlungshärtbarkeit, gute Pigmenthaftung oder geringe oberflächenspannung, führt.

Es wurde nun gefunden, daß heterofunktionelle Diole der Formel

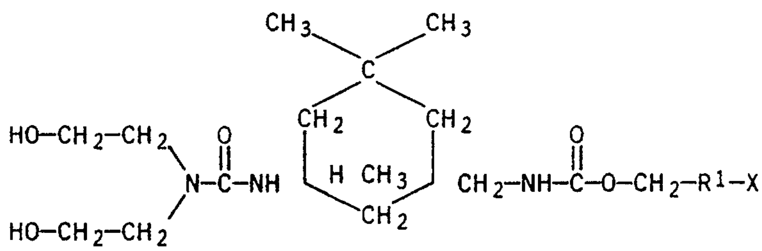

(I)

in welcher

$R^1$ einen geradkettigen oder verzweigten oder cyclischen Rest mit 1 bis 40 Kohlenstoffatomen und mit einem gewichtsmäßigen Anteil der Kohlenstoffatome zwischen 20 und 86 % bedeutet, und

X einen organischen Rest, mit mindestens einer heterofunktionellen Gruppe, wobei die heterofunktionelle Gruppe ausgewählt wird aus der Gruppe

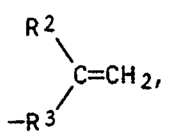 worin $R^2$ gleich H oder $CH_3$ und $R^3$ gleich O, $-O-CH_2-$ oder 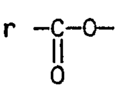 sind,

$-NO_2$, 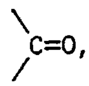, 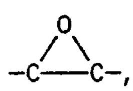, tertiäres oder quartäres Amin

sowie $-SO_3M$, $-OSO_3M$, $-COOM$ und 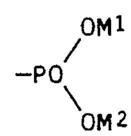

worin M für H, Li, Na, K oder Ammonium und $M^1$ und $M^2$ gleich oder verschieden sein können und jeweils für H, Li, Na, K, Alkyl stehen,

darstellt, zur Lösung der Aufgabe geeignet sind.

Gegenstand der vorliegenden Erfindung sind weiterhin Polyurethanelastomere mit Seitenketten, hergestellt durch Umsetzung

A) eines Polydiols mit einem Molekulargewicht zwischen 400 und 10000,

B) eines Gemisches aus mindestens zwei Diolen und

C) gegebenenfalls einem Tri- oder Polyol mit 3 bis 10 Kohlenstoffatomen mit

D) einem Diisocyanat mit 2 bis 16 Kohlenstoffatomen,

E) sowie gegebenenfalls mit einem Aminoalkohol mit 2 bis 16 Kohlenstoffatomen dadurch gekennzeichnet, daß die Komponente B ein Gemisch aus mindestens einem geradkettigen, aliphatischen Diol (B1) mit 2 bis 10 Kohlenstoffatomen und einem Diol (B2) der Formel (I) besteht, wobei der Anteil des Diols (B2) 0,1 bis 50 % der Gesamtmenge (in Mol) der Komponente B beträgt.

Die Herstellung der erfindungsgemäßen heterofunktionellen Diole gemäß Formel (I) erfolgt in einer an sich bekannten 2-Stufenreaktion. Hierzu wird in der ersten Stufe ein Alkohol der Formel (II)

$HO-CH_2-R^1-X$ (II)

in der $R^1$ und X die obengenannte Bedeutung haben, mit der reaktiveren Gruppe des Isophorondiisocyanats bei Temperaturen zwischen 10 und 60° C gegebenenfalls unter Verwendung eines Katalysators umgesetzt. Anschließend erfolgt ohne Isolierung des Zwischenprodukts die Umsetzung der zweiten Isocyanatgruppe mit Diethanolamin. Die Reaktion kann lösungsmittelfrei oder bei Bedarf in einem Lösungsmittel, vorzugsweise in Tetrahydrofuran erfolgen.

Mit Hilfe dieser erfindungsgemäßen Diole lassen sich Polyurethanelastomere, insbesondere mit Kammstruktur, herstellen, welche sich je nach Art der funktionellen Gruppe durch spezielle Eigenschaften, wie z.B. Strahlungshärtbarkeit, gute Pigmenthaftung oder geringe Oberflächenspannung auszeichnen und hochelastische, auf verschiedenen Substraten gut haftende Oberflächenbeschichtungen ergeben.

Die erfindungsgemäßen Polyurethane können nach den allgemein bekannten Verfahren entweder lösungsmittelfrei oder in Lösung hergestellt werden. Für den Einsatz als Oberflächenbeschichtung werden die Produkte vorzugsweise in Lösung, gegebenenfalls in Gegenwart von Katalysatoren und anderen Hilfsmitteln und/oder Zusatzstoffen hergestellt. In diesem Fall wird in bekannter Weise, z.B. gemäß DE-A 25 00 921, nach Erreichen der gewünschten Endviskosität die Reaktion mit einem Überschuß eines primären und/oder sekundären Monoamins (Komponente E) abgestoppt. Bei der Herstellung in Lösung erweist sich die Kammstruktur der Polymeren als besonders vorteilhaft, da dadurch die Bildung von Wasserstoffbrükkenbildung zwischen einzelnen Polymerketten gestört wird, was zu einer besseren Löslichkeit der Produkte führt.

Als Komponente A (Polydiol) wird ein Polyesterol, Polyetherol oder Polycarbonat mit einem Molekulargewicht von 400 bis 4000, bevorzugt von 700 bis 2500, eingesetzt. Die Polydiole sind zweckmäßigerweise überwiegend lineare Polymere mit zwei endständigen OH-Gruppen. Die Säurezahl der Polydiole ist kleiner als 10 und vorzugsweise kleiner als 3. Die Polyesterole lassen sich in einfacher Weise durch Veresterung von aliphatischen oder aromatischen Dicarbonsäuren mit 4 bis 15 C-Atomen, vorzugsweise 4 bis 8 C-Atomen, mit aliphatischen oder cycloaliphatischen Glykolen, bevorzugt mit 2 bis 20 C-Atomen oder durch Polymerisation von Lactonen mit 3 bis 10 C-Atomen herstellen. Als Dicarbonsäuren lassen sich beispielsweise Glutarsäure, Pimelinsäure, Korksäure, Sebacinsäure, Dodecansäure, Terephthalsäure, Isophthalsäure und vorzugsweise Adipinsäure, Bernsteinsäure und Terephthalsäure einsetzen. Die Dicarbonsäuren können einzeln oder als Gemische verwendet werden. Zur Herstellung der Polyesterole kann es gegebenenfalls vorteilhaft sein, anstelle der Dicarbonsäuren die entsprechenden Säurederivate, wie Carbonsäureanhydride oder Carbonsäurechloride zu verwenden. Beispiele für geeignete Glykole sind Diethylenglykol, Pentandiol, Decandiol-1,10 und 2, 2, 4-Trimethylpentandiol-1,5. Vorzugsweise verwendet werden Ethandiol-1,2, Butandiol-1,5, Hexandiol-1,6 und 2,2-Dimethylpropandiol-1,3, 1,4-Dimethylolcyclohexan, 1,4-Diethanolcyclohexan, 1,4-Diethanolpropan. Je nach den gewünschten Eigenschaften der Polyurethane können die Polyole allein oder als Mischung in verschiedenen Mengenverhältnissen verwendet werden. Als Lactone für die Herstellung der Polyesterole eignen sich z.B. $\alpha,\alpha$-Dimethyl-$\beta$-propiolacton, Butyrolaction und vorzugsweise Caprolacton. Polyetherole sind beispielsweise Polyetrahydrofuran oder Polypropylenoxiddiol, die Polycarbonate sind im allgemeinen auf Hexandiol-1,6-Basis aufgebaut.

Als Komponente B1 werden geradkettige aliphatische Diole mit 2 bis 10 C-Atomen, vorzugsweise 2 bis 6 C-Atome, eingesetzt, so z.B. Ethandiol-1,2, Propandiol-1,3, Butandiol-1,4, Hexandiol-1,6, Pentandiol-1,5, Decandiol-1,10, 2-Methyl-1,3-propandiol, 2-Methyl-2-butyl-1,3-propandiol, 2,2-Dimethyl-1,4-butandiol, 2-Methyl-2-butyl-1,3-propandiol, Hydroxypivalinsäureneopentylglykolester, Diethylenglykol, Triethylenglykol und Methyldiethanolamin, 1,4-Dimethylolcyclohexan, 1,4-Diethanolcyclohexan. Die Diole können einzeln oder als Gemische verwendet werden.

Die Komponente B2 sind die erfindungsgemäßen Diole gemäß Formel (I). Ihr Anteil an der Gesamtmenge der Komponente B (in Mol) beträgt 0,1 bis 50, vorzugsweise 1 bis 30 %.

Als Triole (Komponente C) werden Verbindungen mit 3 bis 10, vorzugsweise 3 bis 6 C-Atomen, angewandt. Beispiele für entsprechende Triole sind Glycerin oder Trimethylolpropan. Geeignet sind auch niedermolekulare Umsetzungsprodukte von z.B. Trimethylolpropan mit Ethylenoxid und/oder Propylenoxid. Das Vorhandensein von Triolen bei der Polyaddition führt zu einer Verzweigung des Endprodukts, was sich,

sofern keine örtliche Vernetzung auftritt, positiv auf die mechanischen Eigenschaften des Polyurethans auswirkt.

Als Polyole (Komponente C) lassen sich beispielsweise Erythrit, Pentaerythrit und Sorbit einsetzen.

Zur Bildung der NCO-gruppenhaltigen Zwischenprodukte werden die unter A, B und C genannten Komponenten mit aliphatischen, cycloalophatischen oder aromatischen Diisocyanten mit 6 bis 30 C-Atomen (Komponente D) umgesetzt. Zu diesem Zweck eignen sich z.B. Verbindungen wie Toluylen-2,4-diisocyanat, m-Phenylendiisocyanat, 4-Chlor-1,3-phenylendiisocyanat, 1,5-Naphthylendiisocyanat, 1,6-Hexanmethylendiisocyanat, 1,4-Cyclohexylendiisocyanat und 1,5-Tetrahydronaphthylendiisocyanat, Diphenylmethandiisocyanat, Dicyclohexylmethandiisocyanat oder Isophorondiisocyanat.

Zur Bildung von Polyurethanen mit OH-gruppenhaltigen Harnstoffgruppen an den Kettenenden wird das NCO-gruppenhaltige Zwischenprodukt aus A bis D mit Aminoalkoholen (Komponenten E) umgesetzt. Diese Aminoalkohole mit 2 bis 16, vorzugsweise 3 bis 6 C-Atomen sind u.a. Monoethanolamin, Methylisopropanolamin, Ethylisopropanolamin, Methylethanolamin, 3-Aminopropanol, 1-Ethylaminobutanol-2, 4-Methyl-4-aminopentanol-2, N-(2-Hydroxyethyl)anilin. Diolamine sind besonders geeignet, da durch ihre Anlagerung am Kettenende die OH-Zahl der Polymeren verdoppelt wird. Als besonders günstig haben sich Diethanolamin und Diisopropanoldiamin erwiesen.

Das Verhältnis der Komponenten A bis D kann von 1,35 bis 13 Mol Diisocyanat je Mol Polydiol bei Verwendung von 0,3 bis 10 Mol des Diols mit 2 bis 20 C-Atomen und 0,05 bis 0,5 Mol Triol variiert werden, wobei die Menge des verwendeten Diols teilweise von dem Molekulargewicht des verwendeten Polydiols abhängt. Aus praktischen Gründen ist es jedoch zweckmäßig, einen Diisocyanatüberschuß von 3 über der zur vollständigen Umsetzung der Reaktionsteilnehmer erforderlichen Menge zu verwenden, so daß das Verhältnis der Zahl der eingesetzten Hydroxylgruppen zur Zahl der Isocyanatgruppen in der Reaktionsmischung 1,03 bis 1,3, vorzugsweise 1,05 bis 1,15 beträgt. Der NCO-Überschuß wird dann stöchiometrisch mit den NH-Gruppen des Aminoalkohols umgesetzt, so daß das Verhältnis der Komponenten (A bis C):D:E 1:1,03 bis 1,3:0,03 bis 0,3, bevorzugt 1:1,05 bis 1,15:0,05 bis 0,15 beträgt.

Beim 2-Stufenverfahren sind je nach Reaktionsbedingungen (Lösungsmittelmenge, Reaktionswärme) zwei unterschiedliche Fahrweisen möglich.

Fahrweise 1: Das Diisocyanat wird mit etwas Lösungsmittel vorgelegt, dann werden die Bausteine A, B, C und gegebenenfalls der Katalysator und die Hilfs- und Zusatzstoffe in Lösungsmittel bei Temperaturen von 20 bis 90 °C, in 0,2 bis 5 Stunden zugegeben. Die Komponenten werden bis zum gewünschten NCO-Gehalt umgesetzt, dann wird in der 2. Stufe der Baustein E zugesetzt.

Fahrweise 2: Bei diesem Verfahren werden alle Ausgangskomponenten A bis D in einem Teil des Lösungsmittels gelöst, so daß die Lösungen mit einem Feststoffgehalt von 15 bis 50 Gew.-% gebildet werden. Anschließend werden die Lösungen unter Rühren auf Temperaturen von 20 bis 90 °C, vorzugsweise von 30 bis 70 °C, gegebenenfalls nach der Katalysatorzugabe, erwärmt. Dann werden die Komponenten bis zum gewünschten NCO-Gehalt umgesetzt, danach wird in der 2. Stufe der Baustein E zugegeben.

Beim 2-Stufenverfahren wird in der ersten Stufe mit einem NCO-Überschuß, gegenüber den Bausteinen A bis C gearbeitet. Bei beiden Fahrweisen ist es möglich, in einem Teil des Lösungsmittels die Reaktion zu beginnen und das restliche Lösungsmittel während oder nach der Reaktion zuzugeben.

Als Lösungsmittel für die Herstellung der Polyurethane werden vorzugsweise cyclische Ether, wie Tetrahydrofuran und Dioxan, und cyclische Ketone, wie Cyclohexanon, verwendet. Selbstverständlich können je nach Anwendungsgebiet die Polyurethane auch in anderen stark polaren Lösungsmitteln, wie Dimethylformamid, N-Methylpyrrolidin, Dimethylsulfoxid oder Ethylglykolacetat gelöst werden. Ebenso ist es möglich, die genannten Lösungsmittel mit Aromaten, wie Toluol oder Xylol und Estern, wie Ethyl- oder Butylacetat, zu mischen.

Als geeignete Katalysatoren zur Herstellung der Polyurethane und für die Vernetzungsreaktion seien beispielhaft genannt: tert. Amine, wie Triethylamin, Triethylendiamin, N-Methyl-pyridin und N-Methylmorpholin, Metallsalze, wie Zinnoctoat, Bleioctoat und Zinkstearat und organische Metallverbindungen, wie Dibutylzinnlaurat. Die geeignete Katalysatormenge ist abhängig von der Wirksamkeit des in Frage kommenden Katalysators. Im allgemeinen hat es sich als zweckmäßig erwiesen, 0,005 bis 0,3 Gewichtsteile für jeweils 100 Gewichtsteile, vorzugsweise 0,01 bis 0,1 Gewichtsteile für jeweils 100 Gewichtsteile Polyurethan zu verwenden.

Die Erfindung sei anhand folgender Beispiele näher erläutert.

Beispiel 1

222 Teile Isophorondiisocyanat wurden in 443 Teile Tetrahydrofuran gelöst und Dibutylzinndilaurat als

Katalysator zugesetzt. Anschließend wurden bei 60 °C 116 Teile Hydroxyethylacrylat zugetropft. Nach Erreichen des theoretischen NCO-Gehalts wurden unter Eiskühlung 105 Teile Diethanolamin zugegeben. Es entstand eine klare 50%ige Lösung. Anhand des IR-Spektrums läßt sich die vollkommene Umsetzung der NCO-Gruppen zeigen. Das Produkt fiel in einer für die Weiterverarbeitung ausreichenden Reinheit an.

Beispiel 2

Es wurde wie in Beispiel 1 beschrieben verfahren, jedoch wurden anstelle des Hydroxyacrylats 564 Teile 1H,1H,2H,2H-Perfluor-1-dodecanol eingesetzt und die Reaktion in 891 Teilen Tetrahydrofuran durchgeführt.

Beispiel 3

Es wurde wie in Beispiel 1 beschrieben verfahren, jedoch wurden anstelle des Hydroxyacrylats 270,5 Teile Stearylalkohol eingesetzt und die Reaktion in 597,5 Teilen Tetrahydrofuran durchgeführt.

Beispiel 4

Es wurde wie in Beispiel 1 beschrieben verfahren, jedoch wurden anstelle des Hydroxyacrylats 88 Teile Hydroxybutanon-2 eingesetzt und die Reaktion in 31 Teilen Tetrahydrofuran durchgeführt.

Beispiel 5

Es wurde wie in Beispiel 1 beschrieben verfahren, jedoch wurden anstelle des Hydroxyacrylats 1400 Teile des Polyethermonoolsulfonats Tegomer® HS-3127 (Th. Goldschmidt AG) eingesetzt und die Reaktion in 1727 Teilen Tetrahydrofuran durchgeführt.

Beispiel 6

In einem beheizbaren Reaktionsgefäß, ausgerüstet mit Rührer und Rückflußkühler, wurden 210 Teile eines Polyesters aus Adipinsäure und 1,4-Butandiol (Molekulargewicht 1000), 5.85 Teile 1,4-Butandiol, 1.34 Teile Trimethylolpropan, 88.6 Teile des Diols gemäß Beispiel 1, 131.25 Teile 4,4′-Diphenylmethandiisocyanat in 1311.12 Teilen Tetrahydrofuran gelöst und auf 55 °C erwärmt. Die Komponenten wurden bis zu einer Endviskosität von 20 Pas (bei 60 °C) umgesetzt und anschließend mit 1769.5 Teilen Tetrahydrofuran auf einen Feststoffgehalt von 12.5 verdünnt. Gleichzeitig wurde die Reaktion durch Zugabe von 3.85 Teilen Diethanolamin abgestoppt. Der K-Wert des gebildeten Polymeren beträgt 56,4, gemessen als 1%ige Lösung in Dimethylformamid.
Oberflächenbeschichtungen aus diesem Lack können durch UV- oder Elektronenstrahlen gehärtet werden.

Beispiel 7

In einem beheizbaren Reaktionsgefäß, ausgerüstet mit Rührer und Rückflußkühler, wurden 200 Teile eines Polyesters aus Adipinsäure und 1,4-Butandiol (Molekulargewicht 1000), 22.95 Teile 1,4-Butandiol, 1.34 Teile Trimethylolpropan, 51.81 Teile des Diols gemäß Beispiel 5, 131.25 Teile 4,4′-Diphenylmethandiisocyanat in 1222.05 Teilen Tetrahydrofuran gelöst und auf 55 °C erwärmt. Die Komponenten wurden bis zu einer Endviskosität von 20 Pas (bei 60 °C) umgesetzt und anschließend mit 1629.4 Teilen Tetrahydrofuran auf einen Feststoffgehalt von 12.5 % verdünnt. Gleichzeitig wurde die Reaktion durch Zugabe von 3.85 Teilen Diethanolamin abgestoppt. Der K-Wert des gebildeten Polymeren beträgt 58, gemessen als 1%ige Lösung in Dimethylformamid.
Das Vergießen der Lösung führt zu gut haftenden, hochelastischen Oberflächenbeschichtungen.

## Ansprüche

1. Heterofunktionelle Diole der Formel (I)

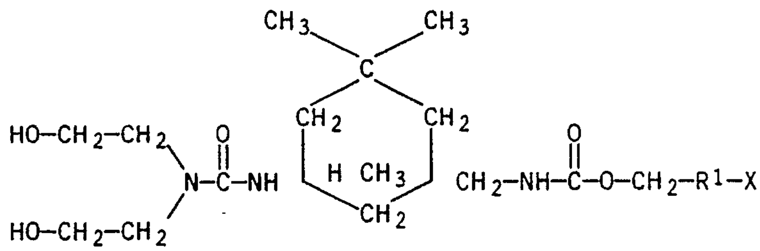

(I)

in welcher
$R^1$ einen geradkettigen oder verzweigten oder cyclischen Rest mit 1 bis 40 Kohlenstoffatomen und mit einem gewichtsmäßigen Anteil der Kohlenstoffatome zwischen 20 und 86 % bedeutet und
X einen organischen Rest, mit mindestens einer heterofunktionellen Gruppe, wobei die heterofunktionelle Gruppe ausgewählt wird aus der Gruppe

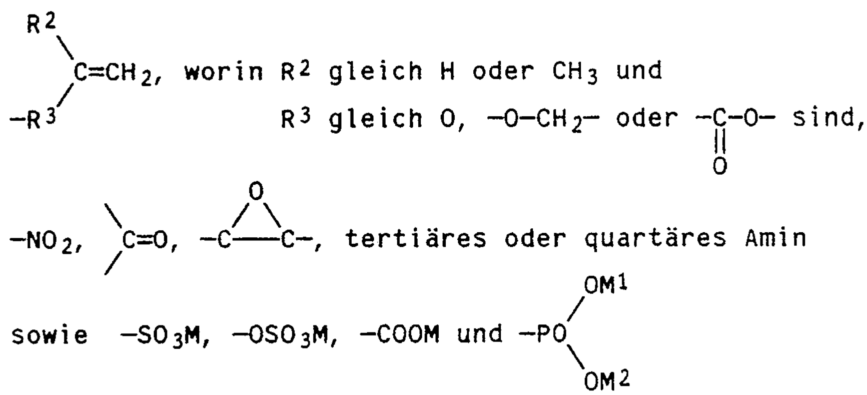

worin $R^2$ gleich H oder $CH_3$ und $R^3$ gleich O, $-O-CH_2-$ oder $-C(=O)-O-$ sind,

$-NO_2$, , , tertiäres oder quartäres Amin

sowie $-SO_3M$, $-OSO_3M$, $-COOM$ und

worin M für H, Li, Na, K oder Ammonium und $M^1$ und $M^2$ gleich oder verschieden sein können und jeweils für H, Li, Na, K, Alkyl stehen,
darstellt.

2. Polyurethanelastomere mit Seitenketten, hergestellt durch Umsetzung
   A) eines Polydiols mit einem Molekulargewicht zwischen 400 und 10000,
   B) eines Gemisches aus mindestens zwei Diolen und
   C) gegebenenfalls einem Tri- oder Polyol mit 3 bis 10 Kohlenstoffatomen mit
   D) einem Diisocyanat mit 2 bis 16 Kohlenstoffatomen,
   E) sowie gegebenenfalls mit einem Aminoalkohol mit 2 bis 16 Kohlenstoffatomen dadurch gekennzeichnet, daß die Komponente B aus einem Gemisch aus mindestens einem geradkettigen, aliphatischen Diol (B1) mit 2 bis 10 Kohlenstoffatomen und einem Diol (B2) der Formel (I) nach Anspruch 1 besteht, wobei der Anteil des Diols (B2) 0,1 bis 50 % der Gesamtmenge (in Mol) der Komponente B beträgt.